# EUROPEAN PATENT APPLICATION

(11) **EP 4 273 231 A1**
(43) Date of publication of application: **08.11.2023**
(21) Application number: 22172735.7
(22) Date of filing: 11.05.2022
(51) Int. Cl.: C12N 5/078, A61K 35/19

(54) **METHOD TO PRODUCE MAMMAL PLATELET CONCENTRATE, MAMMAL PLATELET CONCENTRATE AND USE THEREOF**

(30) Priority: 05.05.2022 PT 2022117961
(71) Applicant: Universidade de Trás-os-Montes e Alto Douro, 5001-801 Vila Real (PT)
(72) Inventor: CLEMENTE PIRES, MARIA DOS ANJOS, 5000-801 VILA REAL (PT); ALVES SOARES, CARLA SOFIA, 4715-349 BRAGA (PT); FERREIRA DE SÁ FARIA, SUSANA MARGARIDA, 4470-338 MAIA (PT); PIRES DE CARVALHO, PEDRO MIGUEL, 3030-509 COIMBRA (PT)
(74) Representative: Monteiro Alves, Inês

(57) **Abstract**

[The present invention discloses an optimized method to produce mammal platelet concentrate (PC) intended for clinical applications and for biotechnological products, such as serum supplementation substitutes.

The present method requires low volume of mammal whole blood, uses specific centrifugal forces and allows the recovery of approximately 5-fold platelet concentration from whole blood baseline.

The PCs produced are intended for autologous and allogenic applications, as individual or pooled PCs. I

## Description

### Technical Field

The present invention describes a method to produce mammal platelet concentrate (PC) intended for the biotechnological area, more specifically, feline platelet concentrate (fePC). The PCs are useful in clinical applications, such as blood transfusions and regenerative therapeutic treatments, as well as in the research area, as serum supplementation substitutes.

### Background Art

Platelet concentrates (PCs) are biological autologous products derived from the patient's blood and consist mainly of supraphysiologic concentration of platelets and growth factors, such as interleukins, having anti-inflammatory and healing enhancing properties.

Recently, human Regenerative Medicine area has gained popularity in both scientific and clinical fields, and PCs / platelet-rich plasma (PRPs) fractions obtained from low volumes of blood has been evoked as therapeutic biological agents, used in the local treatment of different injured tissues. Additionally, the PCs can also be used in cellular cultures as a substitute of xenogenic serum supplements, such as fetal bovine.

When it comes to felines, the ancient clinical interest of feline platelet concentrates (fePCs) is associated to feline blood transfusions wherein the animals are suffering from pathologies such as coagulopathies, platelet dysfunction, and thrombocytopenia.

However, the production of fePC units for hemotransfusion context is considered as an unprofitable process, since (i) several transfusions are generally required in these patients and (ii) the process to produce fePCs is not rentable, since felines are low volume donors and the amount obtained from donation is smaller than the amount necessary to transfuse.

The clinical administration of PCs as a therapeutic option for feline patients, mostly in musculoskeletal disorders (tendinopathies, degenerative joint disease), skin chronic wound healing (chronic wounds, extended traumatic lacerations, fistulas and abscesses) and ophthalmological conditions (such as corneal ulcers and queratitis) has been rising in popularity over the last few years, mainly due to investments performed in the human field

The reasoning for PCs clinical use lies with the fact that platelets contain biologically active peptides that potentiate tissue repair mechanisms, promoting angiogenesis, mitogenesis and extracellular matrix remodeling, also triggering cellular effects in the recruitment of local stem cells, chemotaxis, cell proliferation and differentiation¹⁹.

Regenerative treatments have been cited recently in the state of the art, where felines have received canine sourced platelet-rich hemoderivatives for the treatment of skin and ocular injuries (xenogeneic applications) ^{10,17,18}

The present invention discloses a method to produce mammal platelet concentrate comprising high platelet concentrations and low leukocyte and erythrocyte concentrations.

Such mammal PCs can be produced from differential blood volume collections (partial or major blood collections) and are suitable for autologous and allogenic clinical regenerative applications that require low volumes (local clinical applications) as well as for the production of biotechnological products, such as substitutes for fetal bovine serum in cellular cultures.

The PCs produced according to the method of the present invention are also suitable for the biotechnology industry, contemplating the production of PCs at a larger scale. Moreover, several PCs made from partial or complete blood donations can be pooled at the end of the process, according to the respective animal blood group typing or aleatorily, when the pooled units are going to be lysed (platelet lysate).

The method disclosed in the present invention is new, inventive and significantly different from the methods reported in the state of the art.

Silva et al. (2011) reported a single spin protocol (at 85 g for 6 minutes) to harvest autologous PRPs for clinical settings, resulting in platelet (PLT), leukocyte (WBC) and erythrocyte (RBC) counts of 1021.50 ± 111.55 × 10³ PLT/µL, 6.59 ± 0.78 × 10³ WBC/µL and 2.11 ± 0.36 × 10⁶ %. This methodology provides a PLT-fold increase of 1.65¹⁶.

A recent study, disclosed in Ferrari and Schwartz (2020), tested two commercial systems for the preparation of platelet-rich plasma from felines, allocating 10 cats per system. The tested systems did not achieve 2-5 times platelet concentration from whole blood baseline, and platelet aggregation was pointed as a significant obstacle to consistent generation of feline origin platelet-rich products.

Differently from the present invention, both systems, according to the manufacturer's instructions, were initially described for canine patients, used different anticoagulants and whole blood volumes, and applied different techniques (single spin and double spin, respectively). Finally, the cellular composition, a crucial marker for therapeutic outcome, was significantly different for each system's final platelet-rich plasma fraction, with mean platelet concentration values of 238.00 x10³ PLT/µL (225.00 - 297.00 PLT/µL) and 505.00 ×10³ PLT/µL (166.00 - 1094.00 PLT/µL) for each commercial system. One system recorded the absence of WBC in the produced platelet-rich plasma fraction, while the other a median of 1.35 x10³ WBC/µL, both with similar concentrations of RBC⁷.

Other study conducted by Chun et al. (2020) applying a commercial kit associated with double centrifugation to produce fePCs (1^{st} spin at 3600 RPM for 1 minute and 2^{nd} spin at 3800 RPM for 5 minutes) reported a hematological composition of 777.70 ×10³ (± 561.61) PLT/µL, 4.97 x10³ (± 5.6) WBC/µL) and 0.5 x10⁶ (± 0.39 RBC/µL. This protocol allowed a fePC containing a 2.5-fold increase in platelets from baseline on average⁴.

The method disclosed in the present invention allows the production of mammal PCs with substantial higher platelet concentrations. Moreover, when it regards specifically to fePCs, the PCs produced by the method herein disclosed show a platelet concentration recovery of approximately 5-fold from whole blood baseline, representing a 1.5-fold increase in platelet concentration over the greatest descriptions of platelet-rich plasma cited fractions from felines in the state of the art.

Moreover, the volumes of citrate-based anticoagulants used in the present invention are below the ratio reported in the prior art documents and current guidelines.

### Summary of Invention

The present invention discloses an optimized method to produce mammal platelet concentrate (PCs) comprising the steps of:
(i) providing a whole blood sample from a mammal;
(ii) submitting the whole blood sample to a first centrifugation step to obtain a plasma fraction containing the platelets;
(iii) submitting the plasma fraction containing the platelets, obtained in step (ii), to a second centrifugation step to obtain a mammal platelet concentrate (PC) fraction and a platelet-poor plasma (PPP) fraction; and
(iv) collecting the PC fraction,
   wherein the whole blood sample is homogenized between steps (i) and (ii) and the PC and PPP fractions are homogenized between steps (iii) and (iv) using oscillatory movements

The present method requires low volume of mammal whole blood, uses specific centrifugal forces and allows the obtainment of a mammal platelet concentrate comprising a mean PC volume of 0.45 mL to 0.60 mL, with a median platelet (PLT) concentration of 1300 × 10³ PLT/µL to 1500 × 10³ PLT/µL, a median leukocyte (WBC) concentration of 2.55 × 10³ WBC/µL to 6.60 × 10³ WBC/µL, and a median erythrocyte (RBC) concentration of 0.65 × 10⁶ RBC/µL to 1.45 × 10⁶ RBC/µL.

The PCs produced are intended for autologous and allogenic applications, as individual or pooled PCs, for treating pathologies selected from the group consisting of osteoarthritis, keratitis, corneal ulcers, skin wounds, gingivostomatitis, adjuvant of bone / soft tissue regeneration and as a biotechnological product selected from serum supplementation substitutes.

### Technical Problem

The most recent description techniques for obtaining mammal PCs report the use of commercial kits that were originally designed for humans or canines, and not specifically for felines. Moreover, the PCs produced contains significant lower platelet concentrations when compared to the method disclosed in the present invention.

Furthermore, studies cited in the state of the art perform whole blood analysis in ethylenediamine tetraacetic acid (EDTA) coated tubes, wherein the PCs's heamatological quantification is further performed in citrated samples (comprising a volume of 10 to 16.7 % of citrates anticoagulant). Such measure prevents a realistic comparison between whole blood baseline values and platelet-rich plasma values.

Additionally, some works describing mammal PC preparation express the centrifugation forces in revolutions per minute (rpm) and not in relative centrifugal force (RCF) (g units). The rpm values indicate the speed that the rotor revolves, while RCF values indicate the force applied on the sample, in the centrifuge¹⁴.

### Solution to Problem

Differently from the methods disclosed in the prior art, the present invention relates to a real-time method which is easily performed, and which uses inexpensive materials that are easily available on the market (unlike commercial kits).

The method herein disclosed follows an easily reproducible protocol based on a 2-step centrifugation, which is ready-to-use, and which presents technical details that guarantee the greatest efficiency in the recovery of blood platelets.

Also, the PCs obtained present substantially higher platelet concentrations than reported in the state of the art.

### Advantageous Effects of Invention

The method to produce mammal platelet concentrates (PCs) herein disclosed uses low volume of whole blood as raw material and allows the production of PC samples with high platelet concentrations, being therefore more efficient in the production of PC units.

Also, when compared to the methods disclosed in the prior art, the method herein proposed is optimized and standardized by the use of specific centrifugal forces to concentrate the platelets.

It is also economically advantageous (due to the consumable materials required) and allows the recovery of approximately 5-fold platelet concentration from whole blood baseline.

### Brief Description of Drawings

In order to promote an understanding of the principles according to the modalities of the present invention, reference will be made to the modalities illustrated in the figures and the language used to describe them.

It should also be understood that there is no intention to limit the scope of the invention to the content of the figures and that modifications to the inventive features illustrated herein, as well as additional applications of the principles and embodiments illustrated, which would normally occur to a person skilled in the art having the possession of this description, are considered within the scope of the claimed invention.
[Fig.1] illustrates, respectively, (A) the citrated whole blood inside the respective harvesting syringes after collection; (B) the whole blood from the same donor transferred into a single tube and gently homogenized by tube inversion; (C) the whole blood transferred into a 5 mL polypropylene tube for centrifugation and (D) platelet and part of leukocytes suspended in plasma, being isolated from citrated whole blood after the first centrifugation step.
[Fig.2] illustrates (A) the plasma fraction immediately above the buffy-coat layer is careful collected using a 250 µL pipette and avoiding disturbing the red layer; (B) tubes are placed with an 70° angle; (C) the aspiration of the remainder of the plasma fraction is accomplished drop-by-drop using a 50 µL pipette and (D) the aspiration is complete when the red fraction is being uninterruptedly aspirated.
[Fig.3] illustrates (A) the plasma fraction harvested from the first centrifugation phase; (B) the plasma divided into polypropylene tubes before performing the second centrifugation step; (C) the three-fraction material obtained after the second centrifugation; and (D) the bottom fraction collected, which contains the PRP.
[Fig.4] illustrates the PC fraction, containing mainly platelets and also leukocytes, after the gentle homogenization.

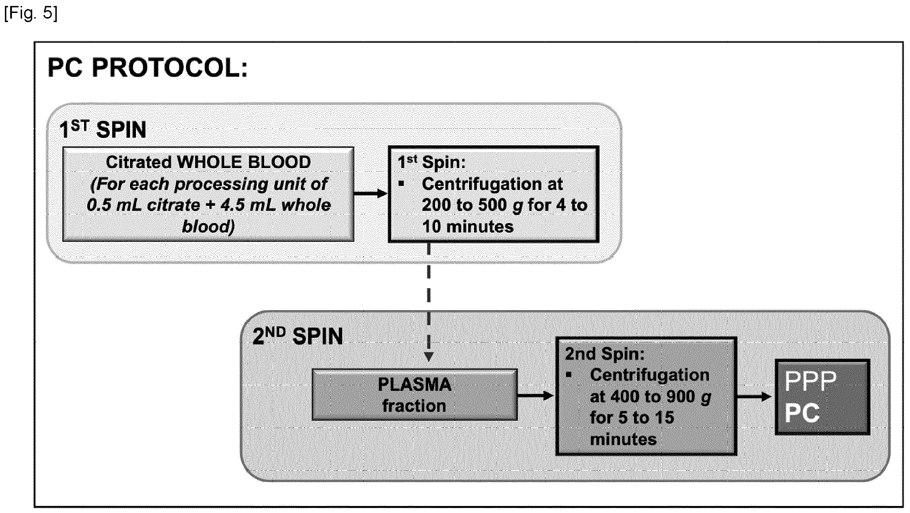

[Fig.5] is a block chart that illustrates the method to produce platelet concentrates of the present invention.
[Fig.6] graphically illustrates the hematological comparison between whole blood and both PC and PPP fractions produced by the method of the present invention.

### Description of Embodiments

The present invention discloses a new and inventive method to produce platelets concentrates, more specifically, mammal platelet concentrate (PC) and comprises the steps of:
(i) providing a whole blood sample from a mammal;
(ii) submitting the whole blood sample to a first centrifugation step to obtain a plasma fraction containing the platelets;
(iii) submitting the plasma fraction containing the platelets, obtained in step (ii), to a second centrifugation step to obtain a mammal platelet concentrate (PC) fraction and a platelet-poor plasma (PPP) fraction; and
(iv) collecting the PC fraction,
   wherein the whole blood sample is homogenized between steps (i) and (ii) and the PC and PPP fractions are homogenized between steps (iii) and (iv).

Between each step, the whole blood sample / PC and PPP fractions are gently homogenized using oscillatory movement in order to avoid platelet activation, hemolysis or the formation of foam. The gentle agitation ensures that the platelets are not activated.

In step (i), a mammal whole blood sample is provided, preferably in the volume range of 4.5 to 45 ml. The whole blood sample volume depends on the centrifuge and the tubes used, the minimal whole blood volume required being 4.5 mL. This volume can be scaled up in multiples of 4.5 mL up to a maximum volume allowed by the blood collection guidelines, which imposes limitations for clinically safe blood collection in order to preserve the health status of the donor animal.

In a preferred embodiment of the present invention, the whole blood sample is collected using a syringe containing a citrate-based anticoagulant selected from the group consisting of citrate-phosphate-dextrose solution with adenine (CPDA) or citrate-phosphate-dextrose (CPD).

The whole blood sample is collected from the mammal selected as blood donors by the veterinary clinician, following the clinical guidelines inherent to this point¹⁵. All the donors should not be under any drug (pharmacological or biological) or vaccine in the previous 15 days before the scheduled blood donation. In a preferred embodiment of the invention, the mammal is a feline or a canine, more preferably a feline.

The whole blood sample is collected from a large caliber vein (the jugular vein is recommended) and should occur immediately after the trichotomy and aseptic cleansing of the region (with chlorhexidine gluconate solution 2 % or similar, followed by ethanol 70 %).

In a preferred embodiment of the invention, the whole blood sample is collected using a citrated syringe having a specific dimension, as recommended by the guidelines for blood collection / donation, preferably a 20G needle butterfly catheter. Such type of instrument prevents the lysis of the cellular elements and the platelet activation and allow the attachment of the collection syringe(s).

The line tube of the collection system is flushed with the anticoagulant solution found within the collection syringe immediately before the venous puncture to avoid the clot formation at the time of the blood harvesting.

The following steps, which involve the preparation and manipulation of the whole blood samples, is performed in a laminar flow chamber / or in a surgical environment, maintaining aseptic and sterile conditions throughout the process.

During the process, the whole blood sample, as well as its derived fractions (plasma, platelet concentrate - PC - and platelet-poor plasma - PPP) are homogenized using gentle oscillatory movements to avoid the formation of small caliber clots and platelet aggregates.

In step (ii), the whole blood sample is submitted to a first centrifugation to obtain a plasma fraction containing the platelets. The centrifugation will allow the recuperation of platelets and part of leukocytes and, at the same time, the separation of the erythrocytes.

In a preferred embodiment of the invention, the first centrifugation step is performed in a range of 200 to 500 g for 4 to 10 minutes, preferably 400 g for 5 minutes. At the end of step (ii), the whole blood sample will be divided in two fractions: the plasma fraction containing the platelets (upper fraction) and the red blood fraction (bottom fraction). The plasma fraction containing the platelets of the whole blood sample is recovered by pipetting, wherein an extraction tube is angled in a range from 45 to 80° in relation to a horizontal plane.

Then, in step (iii) the recovered plasma fraction containing the platelets is again submitted to a second centrifugation step to obtain two fractions: the PC and the PPP fractions. In a preferred embodiment of the invention, the second centrifugation step is performed in a range of 400 to 900 g for 5 to 15 minutes, preferably 800 g for 10 minutes.

At last, in step (iv), the numerical volume of plasma is divided in three equal fractions, wherein the two upper fractions are PPPs (platelet-poor plasma) and the bottom fraction is the material of interest - the PC fraction. The PC fraction is collected by pipetting wherein an extraction tube is angled in a range from 45 to 80° in relation to a horizontal plane and the PC units are ready to be used or may be stored and conserved in accordance with its future use and processing requests.

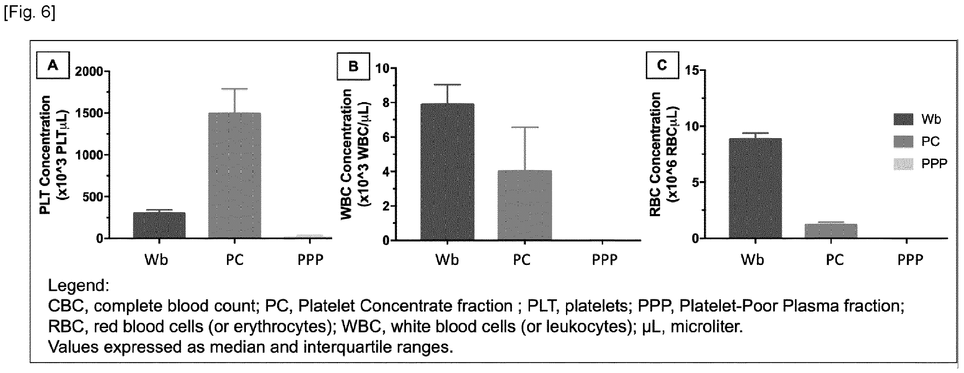

In a preferred embodiment of the invention, the method of the present invention allows the recovery of approximately 5-fold platelet concentration from whole blood baseline (Figure 6).

Starting from a low volume of whole blood sample (of 4.5 mL), this method allows to obtain a mammal platelet concentrate comprising a mean PC volume of 0.45 mL to 0.60 mL, with a median platelet (PLT) concentration of 1300 × 10³ PLT/µL to 1500 ×10³ PLT/µL (blood baseline median concentration = 257.50 × 10³ PLT/ µL), a median leucocyte (WBC) concentration of 2.55 × 10³ WBC/µL to 6.60 × 10³ WBC/µL (blood baseline median concentration = 12.70 × 10³ WBC/µL) and a median erythrocyte (RBC) concentration of 0.65 × 10⁶ RBC/µL to 1.45 × 10⁶ RBC/µL (blood baseline median concentration = 8 ×10⁶ RBC/µL).

The method herein proposed is versatile and can be adapted to produce PCs from a minimal whole blood sample (minor-PCs), small whole blood samples that do not constitute a whole blood sample (partial-PCs) or larger whole blood volume samples (major-PCs or pooled-PCs).

Minor-PC units are obtained from a minimal whole blood sample comprising 4.5 mL of whole blood volume and partial-PC units are obtained from small mammal blood collection that do not constitute a complete blood collection, where the collected blood volume is a multiple of 4.5 mL. Both minor-PC and partial-PC are suitable for autologous applications.

Major-PC units are obtained from a complete mammal blood donation, where a maximal and clinically safe volume of blood is collected from the donor, considering its body weight, as already performed in clinical field and human Blood Banks ^{5,22}. The blood to be collected follows the same methodology applied for blood transfusions and a healthy donor may safely donate 50 - 60 mL of whole blood.

Pooled-PC units are the combination of at least two units of PCs, wherein partial-PCs or major-PCs are pooled at the end of the process, according to the respective blood group typing or aleatorily, when the pooled units are going to be lysed (platelet lysate).

Both minor-PC and partial-PC are suitable for allogenic applications, that implicate the screening of the donors.

The PC obtained are suitable for clinical solicitations in felids (e.g. as a medicinal product, which can be used in the form that it is obtained or transformed). In an alternative embodiment of the present invention, the PC is lyophilized or freeze-dried, being easily reconstituted when needed.

Additionally, the PCs are also useful as raw material to produce biotechnological requests products (e.g. PC as a substitute of fetal bovine serum in cellular culture of felids).

In preferred embodiments of the present invention, the PCs obtained can be used alone (in the present physical form or presentation or other physical or chemical form or presentation) or combined with at least one bioactive agent with pharmacologic action or non-active substances (e.g. adjuvants, delivery vehicles / carriers).

Autologous and allogenic PCs obtained from minor and partial whole blood samples are indicated for the treatment of several pathologies, combined or not with other pharmacological or non-pharmacological products ^{4,6,12,19}.

The pathologies are selected from the group consisting of osteoarthritis, keratitis, corneal ulcers, skin wounds, gingivostomatitis, adjuvant of bone / soft tissue regeneration.

It is important to emphasize that allogenic clinical administrations of PCs should always follow the recommended guidelines regarding to veterinary blood transfusions, implemented in each country / member state, considering the exposition to possible blood-borne infectious / parasitic agents present (the geographical localization should be considered).

Pooled-PCs can be used in the research area or in veterinary clinical applications, preferably for (i) the supplementation of xeno-free cellular cultures from felids's origin; (ii) the supplementation of cellular cultures in clinical area of cellular culture from felids's origin, (iii) the development of novel platelet-based therapeutic products for felids (isolated or combined with other active or non-active ingredients).

### Examples

In an example of the present invention, in order to produce a single fePC unit, a 4.5 mL feline whole blood sample is requested.

The whole blood sample is collected using a 5 mL capacity syringe containing 0.5 mL of citrate-phosphate-dextrose solution with adenine (CPDA) [0.5 mL citrate + 4.5 mL of whole blood].

If two or more fePC units are required from the same donor, the butterfly catheter allows the continuous blood collection by coupling different syringes. The line tube of the collection butterfly system should be flushed with anticoagulant solution immediately before the blood puncture, followed by collection. The proportion of whole blood to be collected and CPDA solution must be respected. Nevertheless, it is mandatory to obtain an additional sample of citrated whole blood sample for each feline donor.

Immediately after the collection, the syringes containing the harvested blood are properly closed and homogenized with gentle inversion of the syringes for 1 minute.

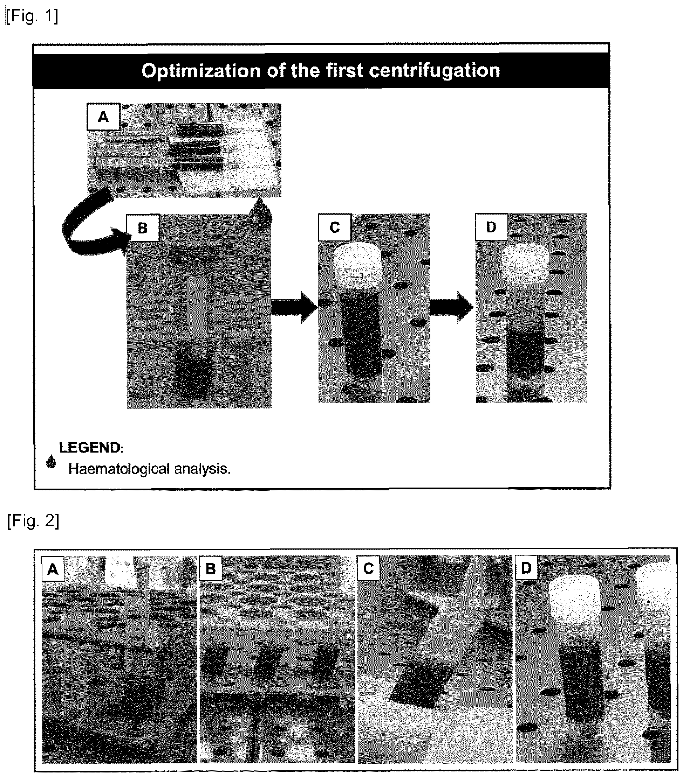

The processing of the whole blood samples is initiated within a maximum of 2 hours after the blood collection. Inside the flow chamber / surgical environment, the collected citrated whole blood is gently transferred into a single polypropylene 50 mL volume tube (30 mm outside diameter, 115 mm length), using slow movements in order to avoid platelet activation and hemolysis (Figure 1A and 1B).

After that, the blood is homogenized by gentle tube inversions for 1 minute and a 0.5 mL citrated whole blood sample is taken for heamatological analysis (complete blood count and CBC, which should be within the normal values range to be used in the production of fePC). Generally, a minimal whole blood sample volume of 0.5 mL is needed for a hematological analysis, but depends on the manufacturer's specification, according to the hematological analyzer used.

Following, the citrated whole blood is homogenized with gentle tube inversions for 1 minute and the blood is then processed in 5 mL aliquots (Figure 1C).

The whole blood samples are centrifuged at 400 g for 5 minutes in conical base polypropylene tubes (57 × 15.3 mm), in a swinging-bucket rotor centrifuge without centrifugal brake and with a deacceleration of 26 seconds, at room temperature (20 - 25 °C).

After the first centrifugation step, the whole blood samples are carefully transferred from the centrifuge to the laminar flow chamber / surgical environment, avoiding disturbing the formed layers (Figure 1D).

The plasma fraction is collected into a new empty polypropylene tube using a 250 µL pipette. The pipette tip is placed immediately above the top line of the upper layer, with a view to prevent it from touching the red fraction. The pipette is maintained in close contact to the tube wall during the aspirations.

Nearby the buffy coat layer, the pipette is slowly removed, and the tubes are gradually and carefully placed in an 70° angle. The aspiration of the remainder of the plasma fraction is accomplished drop-by-drop using a 50 µL pipette, providing a higher volume aspiration and a complete red fraction / plasma separation (Figure 2).

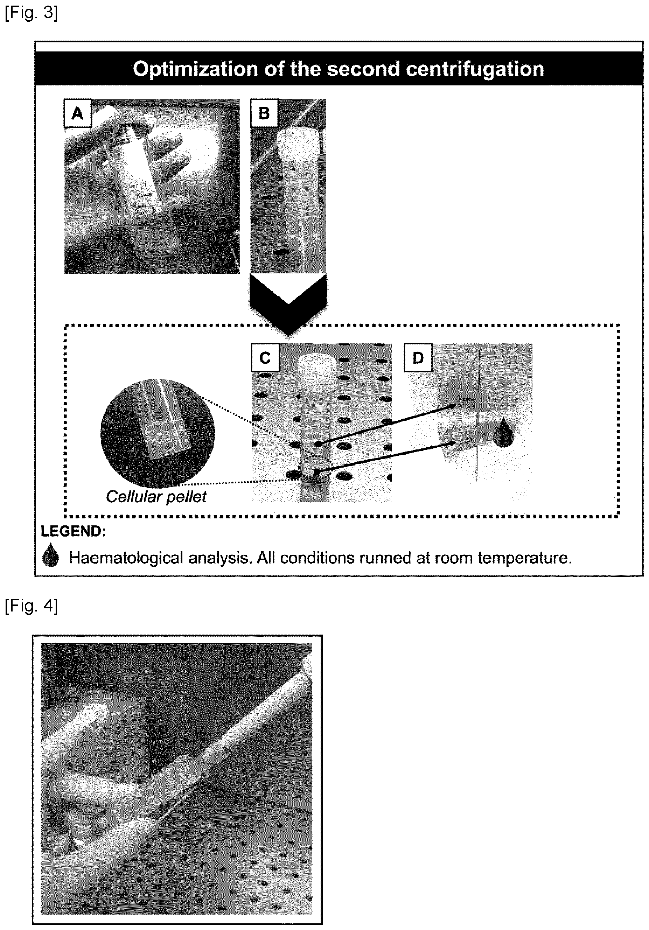

The volume of the plasma fraction obtained depends on the respective donor, being influenced, for example, by the hydration status and the hematocrit of the donor. The plasma fraction is homogenized with gentle tube inversions for 1 minute. If more than one fePC is being produced, all plasma fractions are combined in a single polypropylene tube and the total plasma volume is recorded (Figure 3A). The total volume of these two or more fePCs now united at this step, should occupy 50 to 80% of the polypropylene tube volume capacity, being used for the homogenization of the fePCs.

Next, gently mixing of the plasma fraction is performed for 30 seconds by tube inversion movements before transferring the plasma to a specific conical base polypropylene tube of 57 × 15.3 mm, to be submitted to a second centrifugation. The volume is measured and recorded and if more than one fePC is being produced, the plasma volume is once again divided in equal volumes.

One processing unit is centrifuged in a polypropylene tube of 57 × 15.3 mm with 5 mL capacity (Figure 3B). The plasma is submitted to a second centrifugation spin at 600g to 800g for 10 minutes (without centrifugal brake, and with a deacceleration of 26 seconds, as performed for the first centrifugation).

At the end of the second spin, the plasma is now divided in three equal fractions (Figure 3C): the two upper fractions are PPPs (platelet-poor plasma) and the bottom fraction is the material of interest - the PC fraction (Figure 3D).

The PPP fractions are transferred into a new polypropylene tube (57 × 15.3 mm) and can be conserved at -20 °C or -80 °C, as a raw material (Figure 3D).

The PC fraction (Figure 3D) is homogenously mixed for 30 seconds using a 250 µL pipette and slow up-and-down movements, to avoid the formation of protein foaming (Figure 4). If more than one unit of fePC unit is processed from the same donor, all fePCs are combined into a new polypropylene tube.

At the end of the method, a PC fraction is collected for a hematological analysis (for quality control issues). The FePC units are then ready to be used or may be stored and conserved in accordance with its future use and processing requests, i.e., at refrigeration temperatures (4 °C) when its use is within the following 7 days, or at freezing temperatures (at -20 °C or -80 °C), if intended to be used within 1 year or 5 years, respectively.

The heamatological quantification of the fePCs are shown on Table 1 below:

| F racti on | Hematological Parameter (units) | Median Concentration |
|---|---|---|
| Whole blood | Hematocrit (%) | 29.5 (27.60-33.60) |
| | PLT (×10³/µL) | 297.50 (149.00-340.80) |
| | RBC (×10⁶/µL) | 8.65 (8.12-9.39) |
| | WBC (×10³/µL) | 7.88 (5.41-9.05) |
| PC | PLT (×10⁶/µL) | 1492.00 (890.00-1791) |
| | RBC (×10⁶/µL) | 1.20 (0.66-1.44) |
| | WBC (×10³/µL) | 4.01 (2.55-6.58) |
| PPP | PLT(×10³/µL) | 13.00 (7.50-39.00) |
| | RBC(×10⁶/µL) | 0.01 (0.00-0.01) |
| | WBC (×10³/µL) | 0.00 (0.00-0.01) |
| Legend: | | |
| PC, Platelet Concentrate; PLT, platelets; PPP, Platelet-Poor Plasma; RBC, red blood cells (or erythrocytes); WBC, white blood cells (or leukocytes); µL, microliters. | | |
| Values expressed as median and interquartile ranges. | | |

The subject matter described above is provided as an illustration of the present invention and, therefore, should not be construed to limit it. The terminology employed for the purpose of describing preferred embodiments of the present invention should not be restricted to them.

As used in the description, defined and indefinite articles, in their singular form, are intended for interpretation to also include plural forms, unless the context of the description explicitly indicates otherwise.

Undefined articles "one" should generally be interpreted as "one or more", unless the meaning of a singular modality is clearly defined in a specific situation.

It will be understood that the terms "understand" and "include", when used in this description, specify the presence of characteristics, elements, components, steps and related operations, but do not exclude the possibility of other characteristics, elements, components, steps and operations as well contemplated.

As used throughout this patent application, the term "or" is used in an inclusive sense rather than an exclusive sense, unless the exclusive meaning is clearly defined in a specific situation. In this context, a phrase of the type "X uses A or B" should be interpreted as including all relevant inclusive combinations, for example "X uses A", "X uses B" and "X uses A and B".

In addition, the articles "a" and "an" as used in this application and the appended claims should generally be construed to mean "one or more" unless specified otherwise or clear from the context to be directed to a singular form.

All changes, provided they do not modify the essential characteristics of the following claims, must be considered within the scope of the protection of the present invention.

### Citation List

Here follows the list of citations:

### Non Patent Literature

1. Alio JL, Rodriguez AE, Abdelghany AA, Oliveira RF. Autologous Platelet-Rich Plasma Eye Drops for the Treatment of Post-LASIK Chronic Ocular Surface Syndrome. J Ophthalmol. 2017; 2017:1-6.
2. Astori G, Amati E, Bambi F, et al. Platelet lysate as a substitute for animal serum for the ex-vivo expansion of mesenchymal stem/stromal cells: present and future. Stem Cell Res Ther. 2016; 7:1-8.
3. Canapp SO, Canapp DA, Ibrahim V, Carr BJ, Cox C, Barrett JG. The Use of Adipose-Derived Progenitor Cells and Platelet-Rich Plasma Combination for the Treatment of Supraspinatus Tendinopathy in 55 Dogs: A Retrospective Study. Front Vet Sci. 2016; 3:61.
4. Chun N, Canapp S, Carr BJ, Wong V, Curry J. Validation and Characterization of Platelet-Rich Plasma in the Feline: A Prospective Analysis. Front Vet Sci. 2020 Aug 11; 7:5-7.
5. European Directorate for the Quality, of Medicines & HealthCare (EDQM) C of E. Guide to the preparation, use and quality assurance of blood components. Europe C of, ed. Strasbourg, France: Council of Europe; 2017.
6. Farghali HA, AbdElKader NA, AbuBakr HO, et al. Corneal Ulcer in Dogs and Cats: Novel Clinical Application of Regenerative Therapy Using Subconjunctival Injection of Autologous Platelet-Rich Plasma. Front Vet Sci. 2021 Mar 18; 8:1-17.
7. Ferrari JT, Schwartz P. Prospective Evaluation of Feline Sourced Platelet-Rich Plasma Using Centrifuge-Based Systems. Front Vet Sci. 2020 Jun 12; 7:1-6.
8. Fitzpatrick J, Bulsara MK, McCrory PR, Richardson MD, Zheng MH Analysis of Platelet-Rich Plasma Extraction: Variations in Platelet and Blood Components Between 4 Common Commercial Kits. Orthop J Sport Med. 2017; 5:1-8.
9. Gato-Calvo L, Magalhaes J, Ruiz-Romero C, Blanco FJ, Burguera EF. Platelet-rich plasma in osteoarthritis treatment: Review of current evidence. Ther Adv Chronic Dis. 2019; 10:1-18.
10. Gemignani F, Perazzi A, Iacopetti I. Use of canine sourced platelet-rich plasma in a feline contaminated cutaneous wound. Can Vet J. 2017; 58:141-144.
11. Hewitt CJ, Lee K, Nienow AW, Thomas RJ, Smith M, Thomas CR. Expansion of human mesenchymal stem cells on microcarriers. Biotechnol Lett. 2011 Nov 17; 33:2325-2335.
12. Kim SE, Lee M-K, Seo K. Clinical Application of Serum Eye Drops for Herpetic Keratitis in Cats : A Pilot. Intern J Appl Res Vet Med. 2018; 16:221-225.
13. Kuffler D. Variables affecting the potential efficacy of PRP in providing chronic pain relief. J Pain Res. 2018 Dec; Volume 12:109-116.
14. Miron R, Choukroun J, Ghanaati S. Controversies related to scientific report describing g-forces from studies on platelet-rich fibrin: Necessity for standardization of relative centrifugal force values. Int J Growth Factors Stem Cells Dent. 2018; 1:80.
15. Pennisi MG, Hartmann K, Addie DD, et al. Blood transfusion in cats. J Feline Med Surg. 2015 Jul 22; 17:588-593.
16. Silva RF, Rezende CMF, Paes-Leme FO, Carmona JU. Evaluación del método del tubo para concentrar plaquetas felinas: estudio celular. Arch Med Vet. 2011; 43:187-190.
17. Simona D Pietro, Chiara C, Francesca A, Rosario P, Giusi V, Elisabetta G. Platelet Rich Plasma Eye Drops: Preparation, Storage and Clinical Use in Dogs and Cats. Preliminary Results. Arch Vet Sci Technol. 2017 Feb 10; 1:1-4.
18. Soares CS, Dias IR, Pires MA, Carvalho PP. Canine-Origin Platelet-Rich Fibrin as an Effective Biomaterial for Wound Healing in Domestic Cats: A Preliminary Study. Vet Sci. 2021 Sep 30; 8:213.
19. Soares CS, Babo PS, Reis RL, Carvalho PP, Gomes ME. Platelet-Derived Products in Veterinary Medicine: A New Trend or an Effective Therapy? Trends Biotechnol. 2021 Mar; 39:225-243.
20. Sundman E, Cole B, Karas V, et al. The Anti-inflammatory and Matrix Restorative Mechanisms of Platelet-Rich Plasma in Osteoarthritis. Am J Sports Med. 2014; 42:35-41.
21. Tate K, Crane D. Platelet rich plasma grafts in musculoskeletal medicine. J Prolother. 2010; 371-376.
22. Taylor S, Spada E, Callan MB, et al. 2021 ISFM Consensus Guidelines on the Collection and Administration of Blood and Blood Products in Cats. J Feline Med Surg. 2021 May 26; 23:410-432.
23. Torricelli P, Fini M, Filardo G, et al. Regenerative medicine for the treatment of musculoskeletal overuse injuries in competition horses. Int Orthop. 2011; 35:1569-1576.
24. Wardrop KJ, Birkenheuer A, Blais MC, et al. Update on Canine and Feline Blood Donor Screening for Blood-Borne Pathogens. J Vet Intern Med. 2016; 30:15-35.
25. Wu TE, Chen CJ, Hu C-C, Cheng C-K. Easy-to-prepare autologous platelet-rich plasma in the treatment of refractory corneal ulcers. Taiwan J Ophthalmol. 2015 Sep;5:132-135.

## Claims

1. A method to produce mammal platelet concentrate **characterized in that** it comprises the steps of:
(i) providing a whole blood sample from a mammal;
(ii) submitting the whole blood sample to a first centrifugation step to obtain a plasma fraction containing the platelets;
(iii) submitting the plasma fraction containing the platelets, obtained in step (ii), to a second centrifugation step to obtain a mammal platelet concentrate (PC) fraction and a platelet-poor plasma (PPP) fraction; and
(iv) collecting the PC fraction,
wherein the whole blood sample is homogenized between steps (i) and (ii) and the PC and PPP fractions are homogenized between steps (iii) and (iv) using oscillatory movements.

2. The method, according to claim 1, wherein, in step (i), a small volume of the whole blood sample is provided, preferably in the range of 4.5 to 45 mL, provided that the collected blood volume is a multiple of 4.5 mL.

3. The method, according to claim 1 or 2, wherein the whole blood sample is collected using a syringe containing a citrate-based anticoagulant, wherein the citrate-based anticoagulant is selected from the group consisting of citrate-phosphate-dextrose solution with adenine or citrate-phosphate-dextrose.

4. The method, according to claim 1, wherein, in step (ii), the whole blood sample is submitted to a first centrifugation performed in a range of 200 to 500 g for 4 to 10 minutes, preferably 400 g for 5 minutes.

5. The method, according to claim 4, wherein, at the end of step (ii), the blood is divided in an upper plasma fraction and a bottom red blood fraction.

6. The method, according to claim 5, wherein the plasma fraction containing the platelets of the whole blood sample is recovered by pipetting, wherein an extraction tube is angled in a range from 45 to 80° in relation to a horizontal plane.

7. The method, according to claim 1, wherein, in step (iii), the recovered plasma fraction containing the platelets is submitted to a second centrifugation step performed in a range of 600 to 800 g for 5 to 15 minutes, preferably 800 g for 10 minutes.

8. The method, according to claim 7, wherein, at the end of step (iii), the a numerical volume of plasma fraction containing the platelets is divided in three equal fractions, the two upper fractions being platelet-poor plasma fractions and the bottom fraction being the mammal platelet concentrate fraction.

9. The method, according to claims 7 or 8, wherein the mammal platelet concentrate is recovered by pipetting, wherein an extraction tube is angled in a range from 45 to 80° in relation to a horizontal plane.

10. The method, according to any of claims 1 or 9, wherein the mammal is a feline or a canine.

11. A mammal platelet concentrate obtained by the method disclosed in any of claims 1 to 10, **characterized in that** it comprises a mean PC volume of 0.45 mL to 0.60 mL, with a median platelet (PLT) concentration of 1300 × 10³ PLT/µL to 1500 × 10³ PLT/µL, a median leukocyte (WBC) concentration of 2.55 × 10³ WBC/µL to 6.60 × 10³ WBC/µL, and a median erythrocyte (RBC) concentration of 0.65 × 10⁶ RBC/µL to 1.45 × 10⁶ RBC/µL.

12. The mammal platelet concentrate, according to claim 11, wherein it is provided in the form that it is obtained for fresh use or as a lyophilized or freeze-dried product.

13. A use of the mammal platelet concentrate as disclosed in any of the claims 11 and 12 **characterized in that** it is in clinical applications, in the form that it is obtained or lyophilized or freeze-dried, alone or combined with at least one bioactive agent with pharmacologic action or non-active substances, for treating pathologies selected from the group consisting of osteoarthritis, keratitis, corneal ulcers, skin wounds, gingivostomatitis, adjuvant of bone / soft tissue regeneration.

14. A use of the mammal platelet concentrate as disclosed in any of the claims 11 and 12 **characterized in that** it is as a biotechnological product selected from serum supplementation substitutes.
